# EUROPEAN PATENT SPECIFICATION

(11) **EP 2 194 876 B1**
(45) Date of publication and mention of the grant of the patent: **30.11.2011**
(21) Application number: 08807794.6
(22) Date of filing: 25.09.2008
(51) Int. Cl.: A61B 6/03

(54) **COMPUTER TOMOGRAPHY APPARATUS**
COMPUTERTOMOGRAPHIEGERÄT
APPAREIL DE TOMOGRAPHIE CALCULÉE PAR ORDINATEUR

(30) Priority: 01.10.2007 EP 07117675
(43) Date of publication of application: 16.06.2010
(73) Proprietor: Philips Intellectual Property & Standards GmbH, 20099 Hamburg (DE); Koninklijke Philips Electronics N.V., 5621 BA Eindhoven (NL)
(72) Inventor: BEHLING, Rolf, K., O., NL-5656 AE Eindhoven (NL)
(74) Representative: Van Velzen, Maaike Mathilde
(86) International application number: PCT/IB2008/053897
(87) International publication number: WO 2009/044313

(56) References cited:
- EP-A- 1 772 100
- WO-A-2006/038145
- WO-A1-2006/135837
- WO-A1-2008/122971
- US-A1- 2004 258 196
- US-A1- 2005 190 878
- US-B1- 6 421 412

## Description

This invention relates generally to a computer tomography (CT) apparatus. Particularly, the invention relates to a computer tomography apparatus having a dual focal spot radiation source and an anti-scatter grid. Further, the invention relates to a method for using such a CT apparatus and to a computer program product adapted for controlling said apparatus.

### TECHNOLOGICAL BACKGROUND

X-rays are commonly used to produce images in a variety of settings, including medical diagnosis. X-rays are electromagnetic radiation of extremely short , wavelength, and high energy. This is true even in the range of energies used for medical diagnosis. When an x-ray encounters an atom of matter, it may be absorbed or deflected. The deflected x-rays make up what is known as scatter radiation. Scatter radiation reduces image resolution and enhances image noise. Taking the field of medical diagnosis as an example, the image will ideally be made by only those x-rays that have passed directly through the patient and are attenuated by the body along the path. At any given point of the image, the quantity of x-rays at that point indicates the degree of absorption of the primary beam in the patient on the line from the x-ray source to the x-ray receptor (e.g., the film). The scattered x-rays arrive at the x-ray film from various angles and places in the body not related to the path from the source to the receptor. The unwanted scattered x-rays cause the image to appear clouded. This reduces the image contrast, and obscures small variations that exist within the body being imaged.

One way to reduce x-ray scatter is through the use of a scatter reduction grid, as known from US 2002/0176537 A1.

To receive images from which an exact location of an object within a body can be determined, it is suggested to use a stereo radiograph together with an anti scatter grid, as known from US 6,222,904 B1.

WO 2006/038145 discloses a CT device according to the preamble of claim 1.

### SUMMARY OF THE INVENTION

An object of the invention is to provide a CT system by means of which images with a high resolution can be received.

This object is solved by the subject matter of the respective independent claim. Further exemplary embodiments are described in the respective dependent claims.

According to an exemplary arrangement, a computer tomography apparatus comprises a first source of radiation with a first focal spot, a second source of radiation with a second focal spot, a detector array having a plurality of detector modules arranged in lines and rows on the detector array, a support structure for holding the first and second source of radiation and the detector array. The support structure is adapted for moving the first and second source of radiation and the detector array in a direction around an object of interest such that during operation at least a portion of the radiation emitted from the first and second source of radiation intersects the object of interest before impinging on the detector array. The first and second source of radiation are arranged on the support structure such that a line between the first and second focal spots intersects the direction at an angle different to n*π/2 with n being a natural number different from zero. The special way of arrangement of the two sources of radiation substantially opposite to the detector array provides the basis for the designated high resolution of the received images.

According to another exemplary embodiment, at least one of the rows and the lines of the detector modules on the detector array of the computer tomography apparatus are arranged with the angle to the direction. Thus, the detector modules are arranged directly opposite the sources of radiation. Thereby, the detection of straight beams of the sources of radiation is easier by means of the correspondingly arranged detector modules.

According to yet another exemplary embodiment, the computer tomography apparatus comprises an anti scatter grid on the detector array. The grid provides a further improved resolution of the images.

These and other aspects of the invention will be apparent from and elucidated with reference to the embodiment described hereinafter.

### BRIEF DESCRIPTION OF THE DRAWING

In the following, the invention will be described by means of an exemplary embodiment with regard to the attached figures.
Figure 1 is a schematic illustration of a single focal radiation imaging.
Figure 2 is a schematic illustration of a ring of a CT system using single focal radiation imaging.
Figure 3 is a schematic illustration of a dual focal radiation imaging.
Figure 4 is an schematic illustration of the arrangement of two sources of radiation according to an exemplary embodiment of the invention.
Figure 5 is a schematic illustration of the arrangement of detector modules at the detector array according to an exemplary embodiment of the invention.
Figure 6 is a schematic illustration of a computer tomography apparatus according to an exemplary embodiment of the invention.
Figure 7 is a diagram showing steps of a method for using a computer tomography apparatus according to an exemplary embodiment of the invention.

### DETAILED DESCRIPTION OF AN EXEMPLARY EMBODIMENT

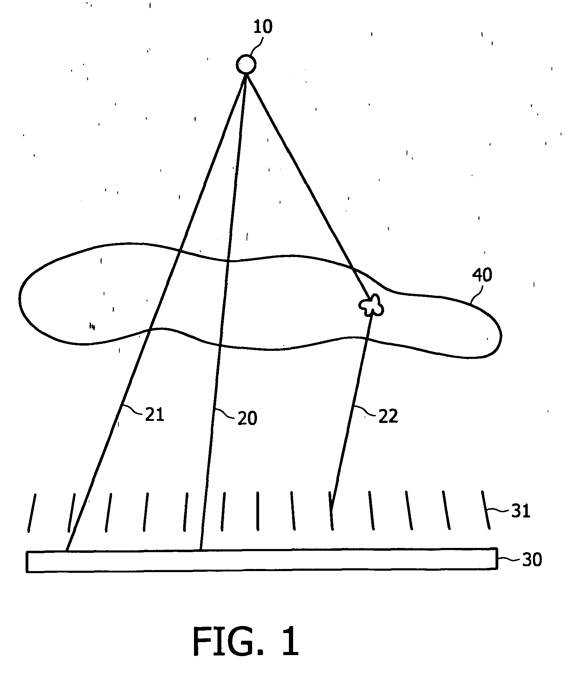

Firstly, the functionality of an anti scatter grid will be described. FIG. 1 shows a single source of radiation 10 and a scatter reduction grid 31. The grid 31 is placed between an object to be imaged, such as a patient 40, and a receptor 30. Ideally, the grid 31 will allow unimpeded passage of an x-ray beam 20 that has come straight from the x-ray source 10 through the patient 40 and will absorb all of the x-ray beams 22 that were scattered by passage through the patient 40.

To improve the effect of the grid 31, it is focused. As seen in FIG. 1, the strips of the focused grid 31 are parallel to each other in their longitudinal direction, but lean toward each other in the direction of x-ray propagation. This allows more primary rays 21 to pass through the grid. However, the focused grid 31 performs well at only one particular distance from the x-ray source 10, since if not at its proper location, the grid 31 will trap many of the primary x-rays as well as the scattered x-rays.

In the focused grid 31, the strips are progressively tilted such that straight lines extended from the points at which the sections intersect with the surface of the grid would intersect at a single point. This point is defined as the focal point of the grid. Thus, focused grids must be used at an appropriate focal distance from the beam source 10, plus or minus an acceptable margin of error.

Further, focused grids may be linear or crossed. A linear grid comprises a single focused anti-scatter grid. A crossed grid comprises two linear grids, one on top of the other or one intersecting the other, such that the strips of one grid are perpendicular to those of the other. Thus, seen from the top, the crossed grids form a kind of a chess board pattern. Crossed grids absorb a significantly higher percentage of the scattered radiation than linear grids, but must be positioned much more carefully relative to the source of the x-ray beam. All grids may also be fixed in position, or moving. Moving grids are attached to a mechanism that is moved as the x-rays pass through the body radiographed. This has the effect of minimizing, in the x-ray image, lines caused by the absorbance of primary x-rays by the grid.

Other technical parameters of the grid are the specific radiopaque and radiolucent materials used, the width of the sections of radiopaque material, the width of the sections of radiolucent material, the height of the grid, the ratio of the height of the grid to the width of the sections of radiopaque material (called the grid aspect ratio), the focal distance of the grid (relevant for focused grids only), and the period of time for which the grid is in motion while the digital sensor plate is being exposed to radiation (relevant for moving grids only). All of these factors determine the extent to which a grid absorbs secondary radiation, the extent to which a grid undesirably absorbs primary radiation, the proper range of focal distances for the grid, the tolerance of the grid for use outside of that range, and the dose of radiation to which the body being radiographed must be exposed in order to generate a useful single radiographic image.

The inherent limitation of two dimensional images is a serious shortcoming of radiography as it exists today. It is desirable for a physician or researcher to know exactly where an object is located within a radiographed body. Although a two dimensional radiographic image presents an internal view of a body, it is difficult to recognize three dimensional structure within a body from a two dimensional radiograph.

There exist several rather complicated techniques for determining three dimensional information within a body. Three dimensional information can be obtained by transmission x-ray microscopy, a combination of an x-ray transmission technique with tomographical reconstruction. This combination allows the obtaining of three dimensional information about the internal microstructure of an object. An internal area is reconstructed as a set of flat cross sections which are used to analyze two and three dimensional morphological parameters. The contrast in the resulting radiographic images is a mixed combination of density and compositional information.

In some cases the compositional information can be separated form the density information with the help of a Computed Tomography scan (CT scan). A CT scan is a medical diagnostic procedure that combines the use of x-rays with computer technology. A series of x-ray beams from many different angles are used to create cross-sectional images of a patient's body. These images are assembled by a computer into a three dimensional picture that can display organs, bones, and tissues in great detail.

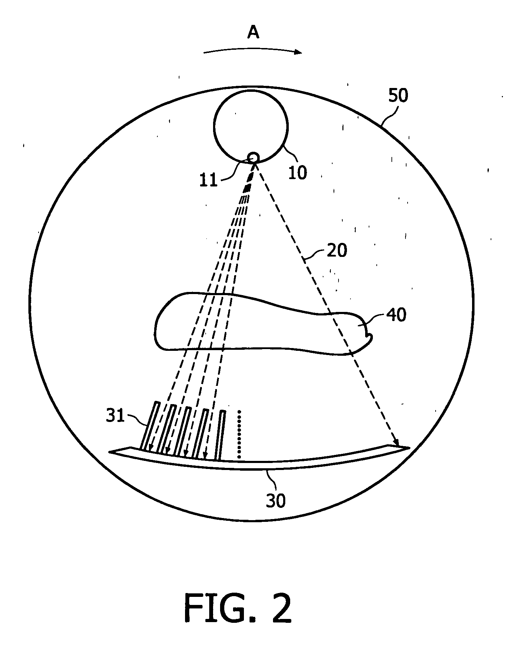

At an application in a computed tomography system, as schematically illustrated in Figure 2, a source 10 for x-rays is placed in a ring 50 opposite a sensor plate 30. X-rays 20 which radiate from the source 10 out from a focus point 11 to a sensor plate 30 which is arranged in the ring of the CT system opposite to said source 10. As described above, there is a grid having inclined lamellas, on the sensor plate 30, wherein the grid 31 is well-disposed with respect to the source 10 for x-rays, so that the x-rays 20 can hit the sensor plate 30 only on the direct way radiating from the focus point 11. At an image in a computer tomography system the radiograph source 10 as , well as the sensor plate 30 are moved along the circumference of the ring now. By this movement several images from different (about 1000 per revolution) subsequent angels are produced of a body which is placed within the ring. These images which are made of a certain cross section within the ring, get combined to build a two-dimensional image of the slice of the body, which is located at the certain cross section, while the radiograph source 10 together with the sensor plate 30 is moved continuously around the body to be x-rayed. Such a system is shown in fig. 2. The radiograph source 10 on the one hand is schematically represented opposite to the sensor plate 30. The rotation of the radiograph source together with the sensor plate within the ring 50 is in the direction of the azimuth A. In fig. 2 the arrow indicates the rotation direction as the said azimuthal direction A. For subsequent positions during the movement of the radiograph source and the sensor plate in azimuthal direction A a new attenuation pattern is created of the body 40 which is located within the ring 50. By means of a computer program system it is possible to combine the continuously successive patterns which were recorded by the sensor plate 30, and reconstruct a two-dimensional image of the body 40. This constitutes a single slice operation. To achieve a three-dimensional analysis of the body, the body 40 is moved gradually in Z direction through the ring. The Z direction in fig. 2 is vertical out of the figure plane.

But, it is a problem of such a procedure that the complete illustration of a body to be x-rayed is very time-consuming. Particularly, if the body shall be represented in detail, the image planes must be taken in short distances one after the other. This means the movement in Z direction can be proceeded only in very small steps.

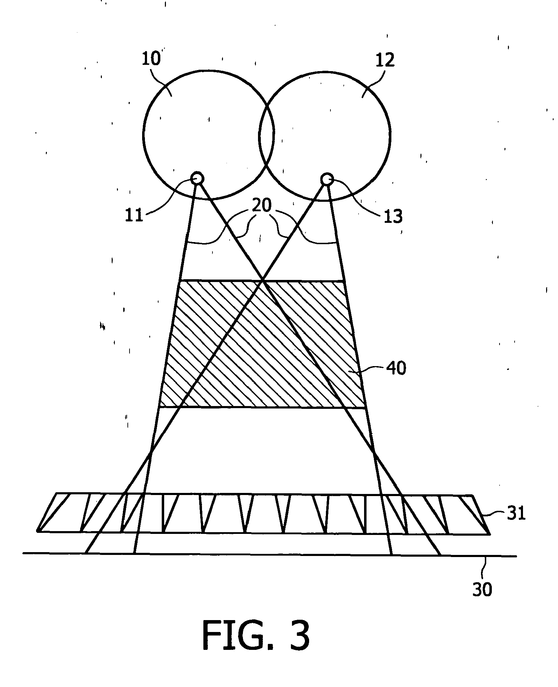

An improvement would be an acquisition of multiple planes in parallel using multiple rows of detector cells (multi-detector CT) mounted adjacent to and behind each other perpendicular to the plane of Fig. 2. Not only one arrangement of detector cells, as shown in Fig. 2, is then illuminated, but e.g. 16, 64 or 128 in parallel. The extension of the detector in axial direction parallel to the patient axis then is largely enhanced. A larger portion of the object can be scanned at the same time. The total acquisition time is reduced considerably. The X-ray beam generated by the source 11 is wider in axial direction ("cone beam") than for a single slice system ("fan beam") as well. A severe basic reconstruction issue arises, however. X-rays now pass in angulated directions through the object. I.e. an angle exists between the plane which is perpendicular to the axial direction and the outer X-ray, which is substantially larger than zero This may create undesired image artifacts ("cone beam artifacts"). Alternatively illuminating the object from two different positions, which are substantially separated in axial direction has been found to reduce the reconstruction and artifact issue.It has been found that a stereo radiographic image can be received by means of two distinct transmitted beams of x-ray which create alternating images on the sensor material positioned on a side of the anti-scatter grid opposite the x-ray sources. Thereupon, it has been suggested to use two sources or radiation together with a scatter grid in a CT system. FIG. 3 is a schematic diagram illustrating a two separate x-ray sources which are positioned on a side of the anti-scatter grid 31 opposite the sensor material 30, a first x-ray source 10 being positioned at a first focal point 11 and a second x-ray source 12 being positioned at a separate, second focal point 13, wherein both x-ray sources are associated with the anti-scatter grid 31.

An imaginary line extends from the left source 10 to the right source 12, i.e. from the left focal point 11 to the right focal point 13, and is parallel to the surface of the sensor material 30.

A focal point associated with the anti-scatter grid 31 is a point at which imaginary straight lines would intersect, the lines being extended from the points at which the progressively angled strips of the anti-scatter grid 31 intersect with the surface of the anti-scatter grid 31. The internal strips of the anti-scatter grid 31 are progressively angled such that two distinct focal points are associated with the anti-scatter grid 31. The focusing of the anti-scatter grid 31 is explained in greater detail later in this section.

X-ray beams 20 are emitted from the left x-ray source 10 and from the right x-ray source 12. The x-ray beams 20 travel through a body 40 being radiographed, to the anti-scatter grid 31. The anti-scatter grid 31 is focused so as to transmit two distinct beams of x-rays to the sensor material 30, beams comprising x-rays traveling in the direction of the x-ray beams from the left x-ray source 10, and other beams comprising x-rays traveling in the direction of the beams from the right x-ray source 12. The sensor material 30 is positioned on a side of the anti-scatter grid 31 opposite the two x-ray sources.

On the sensor material, 30, the two distinct x-ray beams transmitted by the anti-scatter grid 31 reach the sensor material 30 and form two distinct, alternating images thereon, one image associated with the x-ray beams from the left x-ray source 10, and the other image associated with the x-ray beams from the right x-ray source 12.

Practically for use in a CT system, it has been suggested to place radiographic sources directly behind each other so that at a rotation of the sources together with an appropriately bigger sensor plate, several planes of a body can be recorded at the same time. In fig. 3 two radiograph sources are arranged directly besides each other. To get a comparable orientation of the levels which are produced by the two radiograph sources, it is necessary that the two radiograph sources are located in Z direction directly behind each other. This means that from the two sources x-rays are sent out in the same orientation to the body, then penetrate the body, and meet the sensor plate at the same angle and the same time, during a rotation of the radiograph sources together with the sensor plate. As soon as the radiograph sources are not arranged in Z direction directly behind each other, a distortion would arise on'the sensor plate. These distortions would affect the precision of the image reconstruction.

Another aspect of several radiograph sources arranged directly behind each other, is given by the open angle available at radiograph sources. As shown into fig. 3, each of the two represented radiograph sources radiate under a certain angle to the body or an area of interest 40 which is represented by a hatched area. To obtain a complete cover of this area of interest, the radiograph sources must consistently be arranged in a certain distance behind each other. Since radiograph sources are normally of a certain sizes, a combination of two sources directly behind each other in an appropriate distance is difficult, possibly even impossible.

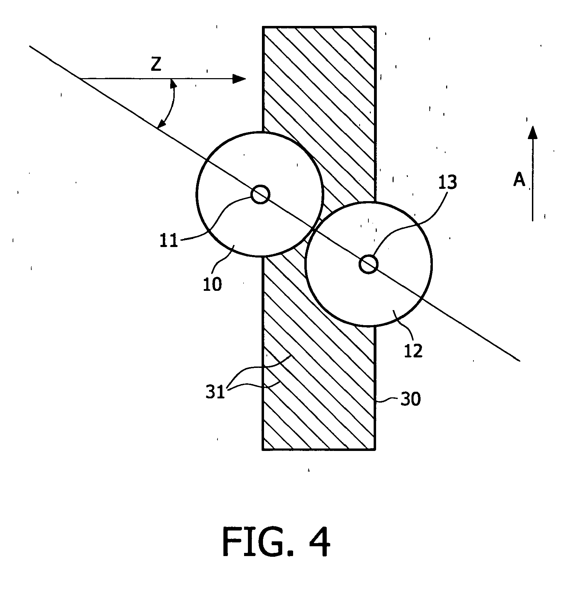

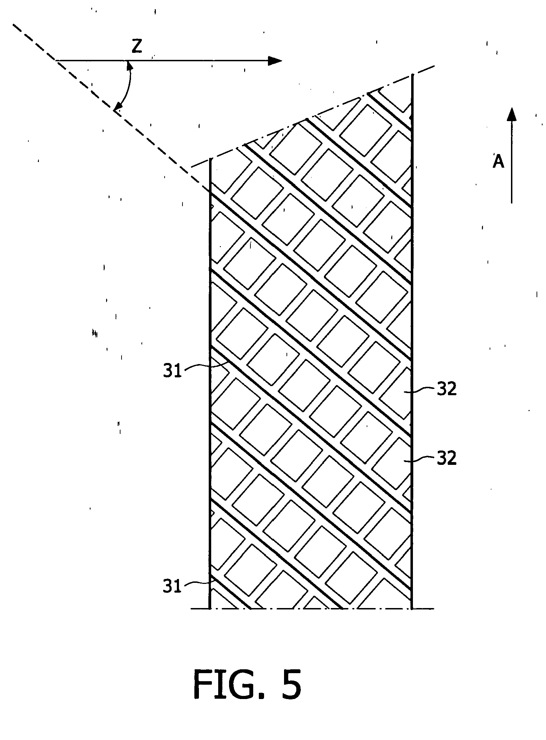

In figure 4, figure 5, the following directions are defined:
as indicated with an arrow, the Z direction runs from the left side of the figure to the right side of the figure;
as also indicated with an arrow, the azimuth direction A runs from the bottom of the side to the top of the side; more precisely, the azimuth direction A runs along the perimeter of the ring of the CT system, wherein the ring of the CT system is positioned vertically to the plane of figures 4 as well as 5. Further, the arrow A indicates the moving direction of the detector array 30. Consequentially, the two sources of radiation 10, 12 will move in the opposite direction in figure 4.

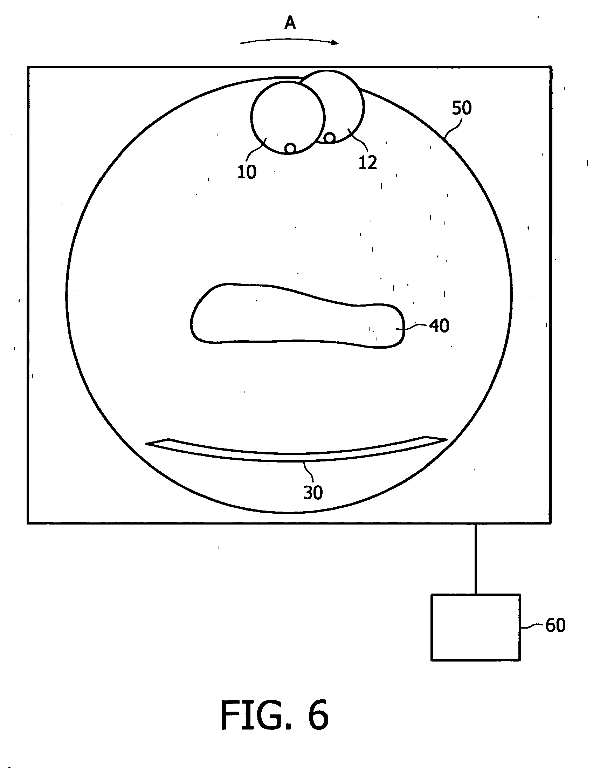

In fig. 4 is shown a detail of a computer tomography (CT) system according to an exemplary embodiment of the invention. The complete computer tomography apparatus according to the invention is shown in figure 6. The CT system comprises a ring 50 which serves as an support structure for holding the two sources of radiation 10, 12. In the ring an object of interest 40 can be placed which, then, can be moved stepwise through the ring 50 of the CT system. While this movement, the hole object or only a part of the object 40 can be radiographed. Thus, a plurality of images are received, wherein each image shows an illustration of one plane through the object of interest, which plane is substantially perpendicular to the z direction. To receive said illustrations, more than one x-ray tube is installed in the ring. In an exemplary embodiment, two x-ray sources 10, 12 are coupled and arranged in a ring 50 of the CT system, wherein the x-ray tubes are movable along the perimeter of the ring, in the azimuthal direction A. The direction of the radiation of each x-ray source is substantially in the plane of the ring 50 and directed substantially to the opposite side of the periphery of the ring. Further, the sources are located behind each other in Z direction as well as behind each other in azimuth direction A. This means that the sources are arranged displaced behind each other in Z direction. In other words, a connection line between the focal points 11, 13 of the two sources 10, 12 is arranged in an determined angle to the Z direction. The angle should be different to n*π/2 with n being a natural number different to null. Thus, the angle is between 0° and 90°, wherein this angle can be measured from the Z direction as well as from the azimuthal direction A. According to an exemplary embodiment, the angle is between 10° and 70° measured from the z direction, particularly between 40° and 60°. According to another exemplary embodiment, the angle is approximately 55° measured from the z direction.

Such an arrangement has the advantage that the sources can be located relative to each other with a small distance in Z direction, though using known x-ray tubes. Thus, there is no need to design new x-ray tubes, since the installation spaces of the used tubes do not intersect each other. A further advantage is that the two x-ray tubes provide a stereoscopic view of the radiographed body, which results in a improved resolution of the received images.

In fig. 4 beneath the sources of radiation, i.e., in the direction in which the sources radiate, a detector array 30 is arranged, on which a grid 31 is located, wherein the grid comprises lamellas. In other words, looking from the sources 10; 12 in the direction of the detector array 30, the lamellas of the grid 31 are arranged corresponding to the arrangement of the sources, i.e. angulated to the z direction. As shown in fig. 4, substantially parallel to the connection line between the two focal points I 1, 13, lamellas are located on the detector array 30. The lamellas are orientated in the direction to the sources of radiation. In particular, the edges of the lamellas, which are parallel to the connecting line between the focal points of the sources, face the focal points 11, 13 of the sources 10, 12.

To fit into the ring of the CT system, the detector array 30 is curved along the perimeter of the ring 50, as illustrated in figure 6. According to the curvature of the ring, the lamellas of the grid are each mounted on the detector array and are each orientated in the direction to the opposite side of the ring, i.e. to the position at which the sources of radiation are located. Thus, the lamellas are inclined relative to each other.

On the detector array 30, there are arranged several detector modules 32. The detector modules are located in rows which extend parallel to the angulated lamellas. The rectangular detector modules 32 are arranged as shown in fig. 5. Therefore, the modules form rows, wherein the rows are angularly orientated to the z direction. On the other hand, the said rows lay side by side on the detector array 30 so that the detector array is completely covered by detector modules 32. Each row of detector modules is located between two lamellas. Since the connection line of the two sources of radiation is above said row, i.e. in the ring 50 opposite to said row, each detector module of said row can detect radiation of both tubes without shadowing effects from any of the lamellas. Thus, an optimal imaging of the body to be radiated, can be achieved. The achieved images will have an improved resolution without any artefacts. The processing of the data sensed by the detector modules, will be executed by means of a computer program product, i.e. a processor unit 60, further having a device to monitor the achieved images.

As another exemplary embodiment, the detector modules at the edges of the detector plate can be inclined so that a radiation beam coming from one of the x-ray tubes will be substantially perpendicular to the upper face of the detector module. Consistently, the detector plate will be curved in z direction, in addition to the curvature along the perimeter of the ring.

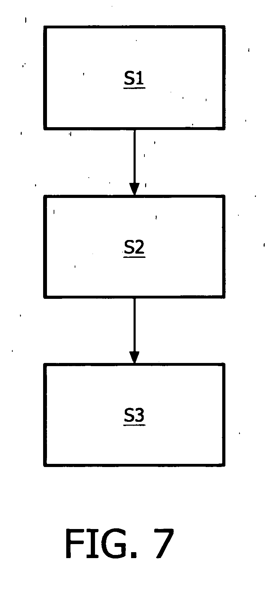

As denoted in figure 7, the computer tomography apparatus can be used in the following steps. Firstly, in step S1, the object of interest is located in the ring of the CT system, i.e. between the sources of radiation and the detector array. In step S2, data will be collected at an angle to the azimuthal direction, by means of the at an angle arranged detector modules. In step S3, images will be generated from the collected data, by means of the processor unit of the CT system.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A computer tomography apparatus comprising:
a first source of radiation with a first focal spot,
a second source of radiation with a second focal spot,
a detector array having a plurality of detector modules arranged in lines and rows on the detector array,
a support structure for holding the first and second source of radiation and the detector array,
a grid with lamellas, said grid located on the detector array, ,
wherein the support structure is adapted for moving the first and second source of radiation and the detector array in a direction around an object of interest such that during operation at least a portion of the radiation emitted from the first and second source of radiation intersects the object of interest before impinging on the detector array,
wherein the first and second sources of radiation are arranged on the support structure such that a line between the first and second focal spots intersects the direction at an angle
wherein the lamellas of the grid are arranged substantially parallel to the line between the first and second focal spot, **characterized in that** the angle is different from n*π/2 with n being a natural number different from zero.

2. The computer tomography apparatus of claim 1, wherein at least one of the rows and the lines of the detector modules on the detector array are arranged with the angle to the direction.

3. The computer tomography apparatus of claim 1 or 2, wherein the grid comprises lamellas arranged parallel to the at least one of the rows and the lines of the detector modules, wherein the edges of the lamellas are facing the sources of radiation.

4. The computer tomography apparatus of any of claims 1 to 3, wherein the support structure is formed as a ring and is adapted for rotating the first and second source of radiation and the detector array in a direction around the object of interest.

5. A method for using a computer tomography apparatus according to any of claims 1 to 4, wherein the method comprises the steps of:
positioning the object of interest between the sources of radiation and the detector array,
collecting data at an angle to the direction, and
generating an image of a cross section of the object'of interest.

6. A computer program product adapted for controlling a computer tomography apparatus according to any of claims 1 to 4, wherein data, collected at an angle'to the direction, are processed to generate an image of a cross section of the object of interest.

## Patentansprüche

1. Computertomographiegerät mit
einer ersten Strahlenquelle mit einem ersten Brennfleck,
einer zweiten Strahlenquelle mit einem zweiten Brennfleck,
einem Detektor-Array mit einer Vielzahl von Detektormodulen, die in dem Detektor-Array in Zeilen und Reihen angeordnet sind,
einer Trägeranordnung zum Aufnehmen der ersten und der zweiten Strahlenquelle und des Detektor-Arrays,
einem Raster mit Lamellen, wobei das genannte Raster auf dem Detektor-Array angeordnet ist,
wobei die Trägeranordnung so ausgelegt ist, dass sie die erste und zweite Strahlenquelle und das Detektor-Array in einer Richtung um ein interessierendes Objekt herum derart bewegt, dass während des Betriebs mindestens ein Teil der von der ersten und der zweiten Strahlenquelle emittierten Strahlung das interessierende Objekt schneidet, bevor er auf den Detektor-Array auftrifft,
wobei die erste und die zweite Strahlenquelle derart auf der Trägeranordnung angeordnet sind, dass eine Linie zwischen dem ersten und dem zweiten Brennfleck die Richtung unter einem Winkel schneidet,
wobei die Lamellen des Rasters im Wesentlichen parallel zu der Linie zwischen dem ersten und dem zweiten Brennfleck angeordnet sind, **dadurch gekennzeichnet, dass** sich der Winkel von n*π/2 unterscheidet, wobei n eine natürliche Zahl ungleich Null ist.

2. Computertomographiegerät nach Anspruch 1, wobei mindestens entweder die Reihen oder die Zeilen der Detektormodule auf dem Detektor-Array unter einem Winkel zu der Richtung angeordnet sind.

3. Computertomographiegerät nach Anspruch 1 oder 2, wobei das Raster Lamellen umfasst, die parallel zu mindestens entweder den Reihen oder den Zeilen der Detektormodule angeordnet sind, wobei die Kanten der Lamellen den Strahlenquellen zugewandt sind.

4. Computertomographiegerät nach einem der Ansprüche 1 bis 3, wobei die Trägeranordnung als Ring ausgebildet und so ausgelegt ist, dass sie die erste und die zweite Strahlenquelle und das Detektor-Array in einer Richtung um das interessierende Objekt herum dreht.

5. Verfahren zum Verwenden eines Computertomographiegerätes nach einem der Ansprüche 1 bis 4, wobei das Verfahren die folgenden Schritte umfasst:
Positionieren des interessierenden Objekts zwischen den Strahlenquellen und dem Detektor-Array,
Erheben von Daten unter einem Winkel zu der Richtung und
Erzeugen eines Bildes eines Querschnitts des interessierenden Objekts.

6. Computerprogrammprodukt, das in der Lage ist, ein Computertomographiegerät nach einem der Ansprüche 1 bis 4 zu steuern, wobei die Daten, die unter einem Winkel zu der Richtung erhoben werden, zur Erzeugung eines Bild des Querschnitts des interessierenden Objekts verarbeitet werden.

## Revendications

1. Appareil de tomodensitométrie, comprenant :
une première source de rayonnement avec un premier foyer,
une seconde source de rayonnement avec un second foyer,
une série de détecteurs possédant une pluralité de modules détecteurs agencés dans des lignes et des rangées sur la série de détecteurs,
une structure de support pour supporter les première et seconde sources de rayonnement et la série de détecteurs,
une grille avec des lamelles, ladite grille étant positionnée sur la série de détecteurs,
dans lequel la structure de support est adaptée pour déplacer les première et seconde sources de rayonnement et la série de détecteurs dans une direction autour d'un objet d'intérêt de sorte que, au cours du fonctionnement, au moins une partie du rayonnement émis à partir des première et seconde sources de rayonnement croise l'objet d'intérêt avant d'être incident sur la série de détecteurs,
dans lequel les première et seconde sources de rayonnement sont agencées sur la structure de support de sorte qu'une ligne entre les premier et second foyers croise la direction à un angle,
dans lequel les lamelles de la grille sont agencées de façon sensiblement parallèle à la ligne entre les premier et second foyers, **caractérisé en ce que** l'angle est différent de n*π/2, n étant un nombre naturel différent de zéro.

2. Appareil de tomodensitométrie selon la revendication 1, dans lequel au moins une des rangées et des lignes des modules détecteurs sur la série de détecteurs est agencée avec l'angle par rapport à la direction.

3. Appareil de tomodensitométrie selon la revendication 1 ou 2, dans lequel la grille comprend des lamelles agencées parallèlement à l'au moins une des rangées et des lignes des modules détecteurs, dans lequel les bords des lamelles font face aux sources de rayonnement.

4. Appareil de tomodensitométrie selon une quelconque des revendications 1 à 3, dans lequel la structure de support présente une forme annulaire et est adaptée pour faire tourner les première et seconde sources de rayonnement et la série de détecteurs dans une direction autour de l'objet d'intérêt.

5. Procédé pour utiliser un appareil de tomodensitométrie selon une quelconque des revendications 1 à 4, dans lequel le procédé comprend les étapes consistant à :
positionner l'objet d'intérêt entre les sources de rayonnement et la série de détecteurs,
collecter des données à un angle par rapport à la direction, et
générer une image d'une section transversale de l'objet d'intérêt.

6. Produit programme d'ordinateur adapté pour commander un appareil de tomodensitométrie selon une quelconque des revendications 1 à 4, dans lequel des données, collectées à un angle par rapport à la direction, sont traitées pour générer une image d'une section transversale de l'objet d'intérêt.
